# DEMANDE DE BREVET EUROPEEN

(11) **EP 0 535 626 A1**
(43) Date de publication de la demande: **07.04.1993**
(21) Numéro de dépôt: 92116721.9
(22) Date de dépôt: 30.09.1992
(51) Int. Cl.: A61K 31/445

(54) **Utilisation de 4-amino-1-(2-pyridyl)pipéridines en tant qu'agents anticonvulsivants et antiépileptiques**

(30) Priorité: 30.09.1991 FR 9112010
(71) Demandeur: SANOFI, F-75008 Paris (FR)
(72) Inventeur: Poncelet, Martine, F-34270 Valflaunes (FR); Guzzi, Umberto, I-20100 Milan (IT)
(74) Mandataire: Gillard, Marie-Louise

(57) **Abrégé**

Les 4-amino-1-(2-pyridyl)pipéridines de formule générale (I) 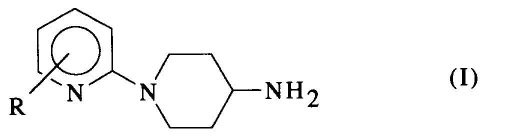
connues en tant qu'agents anorexigènes, ont montré des propriétés pharmacologiques très intéressantes,révélatrices d'une activité anticonvulsivante et antiépileptique.

## Description

La présente invention concerne une nouvelle utilisation en thérapeutique de certaines 4-amino-1-(2-pyridyl)pipéridines.

Le brevet EP-B-0021973 décrit la classe des 4-amino-1-(2-pyridyl)pipéridines de formule générale (I) 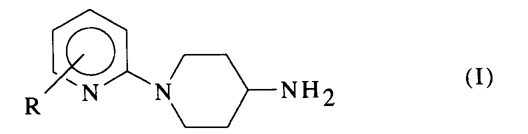
dans laquelle R peut représenter l'hydrogène, un atome d'halogène, un groupe méthyle, trifluorométhyle, (C₁-C₃)alcoxy, trifluorométhoxy, 2,2,2-trifluoroéthoxy, (C₁-C₃)alkylthio, trifluorométhylthio, ou phénoxy ou phénylthio éventuellement substitués par un atome d'halogène, un groupe trifluorométhyle, un groupe (C₁-C₃)alkyle, (C₁-C₃)alcoxy, (C₁-C₃)alkylthio, ou cyano, et de leurs sels pharmaceutiquement acceptables, ainsi qu'un procédé pour leur préparation.

Les composés de formule (I) y sont décrits en tant qu'agents anorexigènes, essentiellement dépourvus d'effets secondaires majeurs tels qu'activité sédative ou excitante, action inhibitrice de l'activité locomotrice et effets cardiovasculaires indésirables.

Parmi les produits de formule (I),le chlorhydrate de 4-amino-1-(6-chloro-2-pyridyl)-pipéridine, décrit dans l'exemple 1 du brevet EP-B-0021973 et nommé CM 57227, a été soumis à tous les tests nécéssaires pour le passage en clinique humaine. Testé chez l'homme à une dose de 10 mg, le produit a été bien toléré.

On a maintenant trouvé que les composés de formule (I) dans laquelle R a la signification donnée ci-dessus, ainsi que leurs sels pharmaceutiquement acceptables, montrent une activité anticonvulsivante très intéressante, à des doses qui ne donnent pas d'activité anorexigène. Les composés de formule (I) et leurs sels d'addition sont donc recommandés dans le traitement des convulsions et de l'épilepsie chez l'homme.

Un premier objet de la présente invention est donc l'utilisation d'au moins un composé de formule (I) 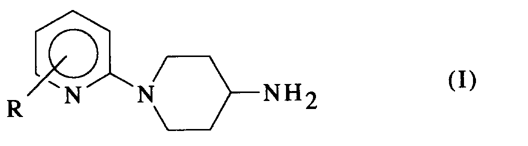
dans laquelle R représente l'hydrogène, un atome d'halogène, un groupe méthyle, trifluorométhyle, (C₁-C₃)alcoxy, trifluorométhoxy, 2,2,2-trifluoroéthoxy, (C₁-C₃)alkylthio, trifluorométhylthio, ou phénoxy ou phénylthio éventuellement substitués par un atome d'halogène, un groupe trifluorométhyle, un groupe (C₁-C₃)alkyle, (C₁-C₃)alcoxy, (C₁-C₃)alkylthio, ou cyano, ou un de ses sels pharmaceutiquement acceptables, pour la préparation d'un médicament destiné à une utilisation en thérapeutique vis-à-vis des convulsions et notamment des convulsions d'origine épileptique.

Dans le texte de la présente demande de brevet, le terme "halogène" identifie l'un des quatre halogènes communs; le fluor, le chlore et le brome étant particulièrement préférés.

Les termes "(C₁-C₃)alkyle", "(C₁-C₃)alcoxy", et "(C₁-C₃)alkylthio" désignent des groupes contenant le résidu d'un hydrocarbure aliphatique saturé contenant 1, 2, ou 3 atomes de carbone, à savoir méthyle, éthyle, propyle et isopropyle.

Les sels pharmaceutiquement acceptables des composés de formule (I) comprennent les sels non toxiques dérivés d'acides minéraux ou organiques salifiant une ou bien les deux fonctions basiques présentes dans les molécules des composés de formule (I) ci-dessus tels que les chlorhydrates, les bromhydrates, les sulfates, les phosphates, les succinates, les tartrates, les fumarates, les maléates, les pamoates, les napsylates, les mésylates, les tosylates, etc.

Une classe préférée de composés de formule (I) comprend les composés de formule (I) dans laquelle R est en position 6- du radical 2-pyridyl, ainsi que leurs sels pharmaceutiquement acceptables.

Une classe encore plus avantageuse comprend les composés de formule (I) dans laquelle R est l'hydrogène, un atome d'halogène, un groupe méthyle ou un groupe (C₁-C₃)alcoxy en position 6, ainsi que leurs sels pharmaceutiquement acceptables.

La 4-amino-1-(6-chloro-2-pyridyl)pipéridine et ses sels pharmaceutiquement acceptables, la 4-amino-1-(6-bromo-2-pyridyl)pipéridine et ses sels pharmaceutiquement acceptables, la 4-amino-1-(6-méthyl-2-pyridyl)pipéridine et ses sels pharmaceutiquement acceptables, la 4-amino-1-(6-méthoxy-2-pyridyl)pipéridine et ses sels pharmaceutiquement acceptables sont des composés particulièrement préférés.

Parmi ceux-ci on préfère encore la 4-amino-1-(6-chloro-2-pyridyl)pipéridine et ses sels pharmaceutiquement acceptables.

"Préféré ou avantageux" se lit ici comme préféré ou avantageux dans la nouvelle utilisation thérapeutique de la présente invention.

Ces composés sont aisément préparés selon la méthode générale décrite dans le brevet EP-B-0021973, en faisant réagir la 2-halopyridine correspondante de formule (II) : 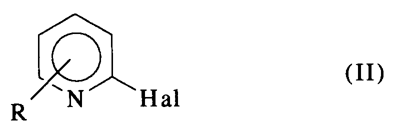
dans laquelle R a la signification donnée ci-dessus et Hal représente un atome d'halogène, avec une 4-aminopipéridine bloquée de formule (III): 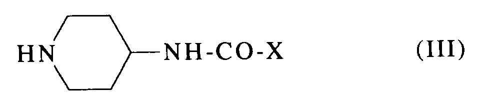
dans laquelle X représente un atome d'hydrogène, ou un groupe (C₁-C₄)alkyle, et en hydrolysant ensuite la 4-amino-1-(2-pyridyl)pipéridine N-acylée ainsi obtenue de formule (IV) : 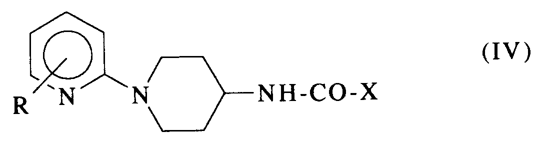
dans des conditions acides ou basiques.

Une description plus detaillée de la méthode générale de préparation des composés (I) ainsi que quelques exemples spécifiques sont contenus dans le brevet européen précité EP-B-0021973.

La nouvelle activité anticonvulsivante des composés de formule (I), a été mise en évidence en évaluant les effets des composés (I) dans le test des convulsions provoquées par électrochoc chez la souris et dans le test des convulsions provoquées par le pentylénététrazole chez la souris selon la méthode décrite par C.S. Goodman, M. Singh Grewal, W.C. Brown et E.A. Swinyard dans J. Pharmacol. Exp. Ther. 108, 168-176, (1953).

Pour ces tests on a utilisé des souris Swiss femelles d'un poids corporel de 22 à 26 g, réparties en lot de 10 animaux chacun. Une semaine avant l'expérimentation, chaque lot de 10 a été mis en cage, gardé dans une pièce à température constante (21°C ± 1°C), en cycle jour/nuit, avec nourriture et boisson à volonté. Les produits de formule (I) à tester ainsi que le pentylénététrazole ont été mis en solution dans de l'eau distillée. Le volume d'injection des produits utilisés a été de 0,4 ml pour 20 g de poids corporel.
· **Convulsions provoquées par le pentylénététrazole.**
   Les composés de formule (I) ont été administrés par voie intrapéritonéale 30 minutes avant l'administration par voie intrapéritonéale de 130 mg/kg de pentylénététrazole (dose nécessaire pour que 80% des souris contrôlées présentent une crise convulsive). On a utilisé des groupes de 10 souris chacun, pour chaque dose [0 mg/kg (contrôle); 3 mg/kg; 10 mg/kg; 30 mg/kg].
· **Convulsions provoquées par électrochoc.**
   Ces crises convulsives ont été induites par électrochoc en utilisant un appareil RACIA pour petits animaux avec électrodes cornéennes. Un choc de 12 volts, appliqué pendant 0,4 secondes, est nécessaire pour que 80% des souris contrôlées présentent une crise convulsive.
   Les composés de formule (I) à tester on été administrés par voie intrapéritonéale 30 minutes avant le choc.

On a utilisé les mêmes doses que dans le test précédent et des groupes de 10 souris pour chaque dose.

Les résultats obtenus dans ces tests montrent que les produits de formule (I) exercent un effet protecteur, vis-à-vis des convulsions, très important avec des DE₅₀ (doses protégeant 50% des animaux) qui sont toujours inférieures à 30 mg/kg.

A titre d'exemple, dans le test des convulsions provoquées par électrochoc la 4-amino-1-(6-chloro-2-pyridyl)pipéridine (le composé de formule (I) dans laquelle R = 6-Cl) montre une DE₅₀ bien inférieure à 3 mg/kg et dans le test des convulsions provoquées par le pentylénététrazole inférieure à 10 mg/kg.

De plus, les composés de formule (I) présentent une faible toxicité, tout-à-fait compatible avec une utilisation thérapeutique.

Sur la base des propriétés ainsi mises en évidence, les composés de formule (I) sont donc recommandés dans le traitement des convulsions et en particulier de l'épilepsie chez l'homme.

Pour l'emploi en tant qu'anticonvulsivants, les composés de formule (I) ainsi que leurs sels pharmaceutiquement acceptables, peuvent être convenablement administrés par voie orale, parentérale, sublinguale, rectale ou transdermique, élaborés sous forme de compositions pharmaceutiques, dans lesquelles ils sont généralement associés à un adjuvant, un diluant et/ou un enrobage compatible et pharmaceutiquement acceptable.

La quantité de principe actif à administrer par jour dépend de la particularité de l'indication thérapeutique, de la gravité des affections à traiter, ainsi que du poids du malade et de la voie d'administration.

En tout état de cause la dose globale chez l'homme varie en général entre 0,05 et 100 mg par jour, par exemple de 0,1 à 50 mg, et plus convenablement de 0,5 à 20 mg par jour.

Les compositions pharmaceutiques, pour l'utilisation des composés de formule (I) en tant qu'agents anticonvulsivants, renfermant au moins un produit choisi parmi les composés de formule (I) et leurs sels d'addition pharmaceutiquement acceptables en association avec un véhicule pharmaceutiquement inerte, peuvent être préparées selon des méthodes usuelles bien connues dans le domaine de la galénique.

Le principe actif peut être incorporé à des excipients habituellement employés dans les compositions pharmaceutiques, tels que le talc, la gomme arabique, le lactose, l'amidon, le stéarate de magnésium, les véhicules aqueux ou non, les corps gras d'origine animale ou végétale, les dérivés paraffiniques, les glycols, les différents agents mouillants, dispersants, ou émulsifiants, les conservateurs, etc.

Pour l'administration par voie orale qui est en tout état de cause la voie d'administration préférée, des formes pharmaceutiques appropriées comprennent les comprimés, les comprimés retard, les dragées, les gélules, les suspensions, les solutions ou encore les liposomes.

En ce qui concerne l'administration intraveineuse, sous-cutanée ou intramusculaire, on recourt à des solutions stériles ou stérilisables, tandis qu'on peut réaliser des suppositoires conventionnels, ou des gélules ou des microclystères pour l'administration rectale.

Pour l'administration transdermique on peut utiliser des patches conventionnels préparés selon des techniques bien connues de l'homme du métier.

Les formes unitaires de dosage pour la nouvelle utilisation thérapeutique comprendront en général de 0,05 à 20 mg, allant de préférence de 0,1 à 10 mg, y compris par exemple le dosage de 0,5 à 5 mg (notamment 0,5; 1; 1,5; 2; 2,5; 3; 3,5; 4; 4,5 et 5 mg) de produit. Ces doses unitaires sont administrées normalement une ou plusieurs fois par jours, de préférence une à trois fois par jour.

Si on le désire, les compositions pharmaceutiques de la présente invention peuvent contenir aussi un ou plusieurs autres médicaments connus et communément utilisés pour les mêmes indications thérapeutiques.

## Revendications

1. Utilisation d'au moins un composé de formule (I) 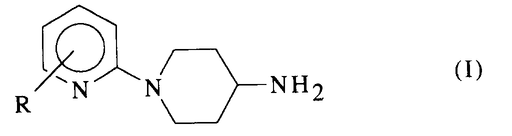 dans laquelle R représente l'hydrogène, un atome d'halogène, un groupe méthyle, trifluorométhyle, (C₁-C₃)alcoxy, trifluorométhoxy, 2,2,2-trifluoroéthoxy, (C₁-C₃)alkylthio, trifluorométhylthio, ou phénoxy ou phénylthio éventuellement substitués par un atome d'halogène, un groupe trifluorométhyle, un groupe (C₁-C₃)alkyle, (C₁-C₃)alcoxy, (C₁-C₃)alkylthio, ou cyano, ou un de ses sels pharmaceutiquement acceptables, pour la préparation d'un médicament à activité anticonvulsivante.

2. Utilisation selon la revendication 1 d'au moins un composé de formule (I) dans laquelle R est en position 6 du radical 2-pyridyle.

3. Utilisation selon la revendication 2 d'au moins un composé de formule (I) dans laquelle R représente un atome d'hydrogène, un atome d'halogène, un groupe méthyle, ou un groupe (C₁-C₃)alcoxy en position 6.

4. Utilisation selon la revendication 3 d'au moins un composé de formule (I) choisi parmi la 4-amino-1-(6-chloro-2-pyridyl)-pipéridine, la 4-amino-1-(6-bromo-2-pyridyl)pipéridine, la 4-amino-1-(6-méthyl-2-pyridyl)pipéridine, la 4-amino-1-(6-méthoxy-2-pyridyl)pipéridine et leurs sels pharmaceutiquement acceptables.

5. Utilisation selon la revendication 4 d'au moins un composé de formule (I) choisi parmi la 4-amino-1-(6-chloro-2-pyridyl)pipéridine et ses sels pharmaceutiquement acceptables.

6. Utilisation selon l'une quelconque des revendications précédentes pour la préparation d'un médicament à activité antiépileptique.
